# EUROPEAN PATENT SPECIFICATION

(11) **EP 2 123 231 B1**
(45) Date of publication and mention of the grant of the patent: **23.01.2019**
(21) Application number: 09006719.0
(22) Date of filing: 19.05.2009
(51) Int. Cl.: A61B 1/005

(54) **Bending tube and medical apparatus**
Biegerohr und medizinische Vorrichtung
Tube de flexion et appareil médical

(30) Priority: 19.05.2008 US 123024
(43) Date of publication of application: 25.11.2009
(73) Proprietor: Olympus Corporation, Tokyo 192-8507 (JP)
(72) Inventor: Dejima, Takumi, Hachioji-shi, Tokyo 192-8507 (JP); Hashimoto, Tatsutoshi, Hachioji-shi, Tokyo 192-8507 (JP)
(74) Representative: von Hellfeld, Axel

(56) References cited:
- EP-A- 0 165 718
- EP-A- 1 967 130
- WO-A-2005/120326
- WO-A-2007/117783
- US-A- 3 060 972

## Description

This application claims priority from U.S. Patent Application No. 12/123,024, filed on May 19, 2008 in the United States Patent and Trademark Office.

### BACKGROUND OF THE INVENTION

### Field of the Invention

The present invention relates to a bending tube capable of bending by operation and a medical apparatus using the bending tube.

### Background Art

Conventionally used endoscopes provided with elongated and flexible insertion section or various medical apparatus including treatment instruments are inserted into the body cavity of a living body for conducting diagnosis or treatment.

In many cases, insertion sections of these medical apparatuses have bending sections capable of bending to permit insertion into a body cavity while following serpentine path, e.g. digestive tract or blood vessel. A commonly used medical apparatus has a transmission member (e.g. a wire) connected to the bending section and extending to an operation section having an angle knob, for example, operated by a user and connected there. The user's operation for the operation section allows the bending section to bend in predetermined directions with a predetermined bending degree.

However, in the conventional medical apparatus, when the user retracts transfer members such as a wire at the operation section, the wire may not be retracted sufficiently due to the restriction of the range of motion of the operation section, and therefore, there is the problem that the bending amount of the bending section may become insufficient. Furthermore, when the operation section of the conventional medical apparatus is constituted to retract the wire sufficiently, the range of motion of the operation section have to be enlarged, and therefore, there is the problem that the design freedom of the medical apparatus is restricted.

International patent application WO 2007/117783 A1 discloses an endoscope comprising a flexible shaft which is insertable in the human body and steerable by control cables. A steering system tension control device is provided for reducing the tension in the control cables. However, the steering system tension control device cannot increase the bending torque to which the shaft is subjected.

A similar medical device is also disclosed in document US 6,236,876.

The present invention is achieved considering the above circumstances, and its object is to provide a bending tube and a medical apparatus which can perform a bending operation of the bending tube easily by the force lower than that of which is conventionally required, or which can perform the bending operation of the bending tube with the manipulated variable smaller than that of which is conventionally required.

### SUMMARY OF THE INVENTION

The invention is defined by the appended claims.

A first aspect of the present invention is a bending tube, provided with a bending section capable of bending, including: a main unit having a lumen; a pair of transfer members having distal ends connected to the bending section; an operation section having proximal ends of the pair of transfer members, the operation section being configured so that retracting operation for retracting one of the pair of transfer members proximally causes the other one of the pair of transfer members to be linked and pushed out distally, wherein the retracting operation applies a predetermined magnitude of pre-tension to the pair of transfer members.

### BRIEF DESCRIPTION OF DRAWINGS

FIG. 1 shows the distal end section of a treatment endoscope provided with a bending tube according to a first embodiment of the present invention in enlarged view.
FIG. 2 shows the arm section of the treatment endoscope in enlarged view.
FIGS. 3A and 3B show an example of conventional bending mechanism provided to the arm section.
FIGS. 4A and 4B show an example of the bending tube of the present invention used for the arm section.
FIG 5A illustrates torque acting on the arm section in the operation as shown in FIG 3B.
FIG. 5B illustrates torque acting on the arm section in the operation as shown in FIG. 4B.
FIG. 6 shows an endoscope provided with a bending tube according to a second embodiment of the present invention.
FIG. 7 shows the mechanism of the operation section of the endoscope in isometric view.
FIG 8 shows the operation section in a pre-tensioned state.
FIG. 9 shows a treatment endoscope provided with a bending tube according to a third embodiment of the present invention.
FIG. 10 shows the operation section of the treatment endoscope in enlarged view.
FIG 11 shows the operation stick of the operation section in cross sectional view.
FIG. 12 shows a first operation shaft and a first wire unit of the operation section.
FIG. 13 shows the first operation shaft and the first wire unit without a cover.
FIG. 14 shows the movement of the first wire unit in a pre-tensioned state.
FIG. 15A shows the joint ring of a bending tube in a modified example of the present invention in isometric view.
FIG. 15B shows the joint ring viewed in the axial line direction.

### PREFERRED EMBODIMENTS

### [First Embodiment]

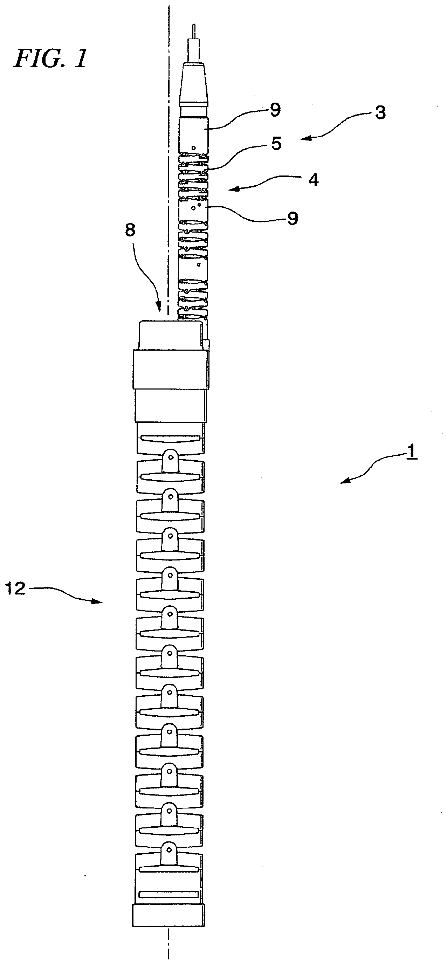

A first embodiment of the present invention will be explained with reference to FIGS. 1 to 5B as follows. FIG. 1 shows an insertion section 2 of a treatment endoscope 1, i.e., the medical apparatus provided with a bending tube of the present embodiment in enlarged view.

The flexible and elongated insertion section 2 has an operation channel, not shown in the drawings, for inserting a treatment instrument therethrough. An arm section 3 is attached to the distal end of the insertion section 2. The arm section 3 has a channel for inserting the treatment instrument therethrough. The channel of the arm section 3 communicates with the operation channel of the aforementioned insertion section 2. An observation device 8 attached to the distal end of the insertion section 2 and movable distally and proximally in a predetermined range permits observation of, for example, a treatment site in the body cavity or of the arm section 3. It should be noted that the number of the attached arm section 3 is not specifically limited.

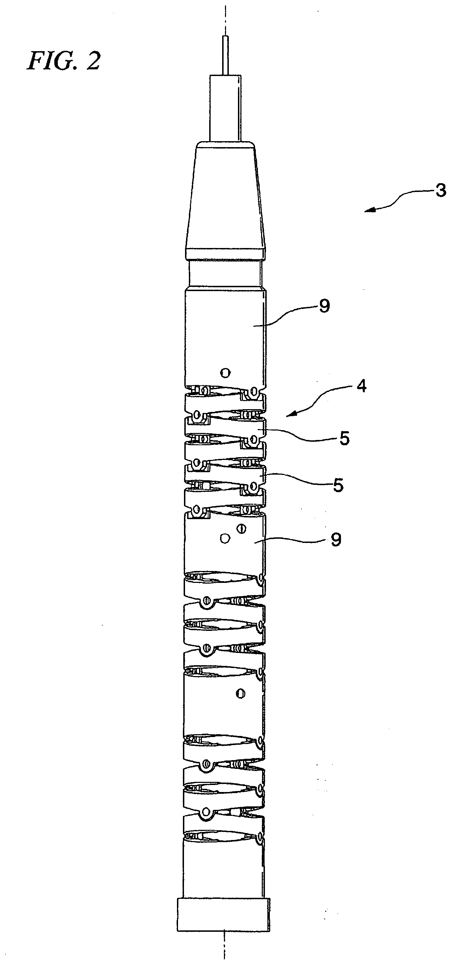

FIG. 2 shows the arm section 3 in an enlarged view. The arm section 3, i.e., the bending tube of the present embodiment, is provided with a substantially cylindrical main unit 9 and a bending section 4 capable of bending by the operation of a user as explained later. The bending section 4 positioned among a plurality of main units 9 are formed by connecting a plurality of joint rings 5 aligned in the axial line direction. Accordingly, the main unit 9 and the joint rings 5 form the aforementioned channel in the lumen of the arm section 3.

A pair of wires, not shown in the drawing, for operating the bending section 4 are disposed to face with each other and to place the axial line of the connected joint rings 5 therebetween. The distal end of each wire fixed to the main unit 9 connected to the most distal end of the bending section 4 extends to a proximally-provided operation section, which will be explained later, while passing through an insertion tube, not shown in the drawing, provided in each joint ring 5. In addition, the proximal end of each wire is connected to the operation section.

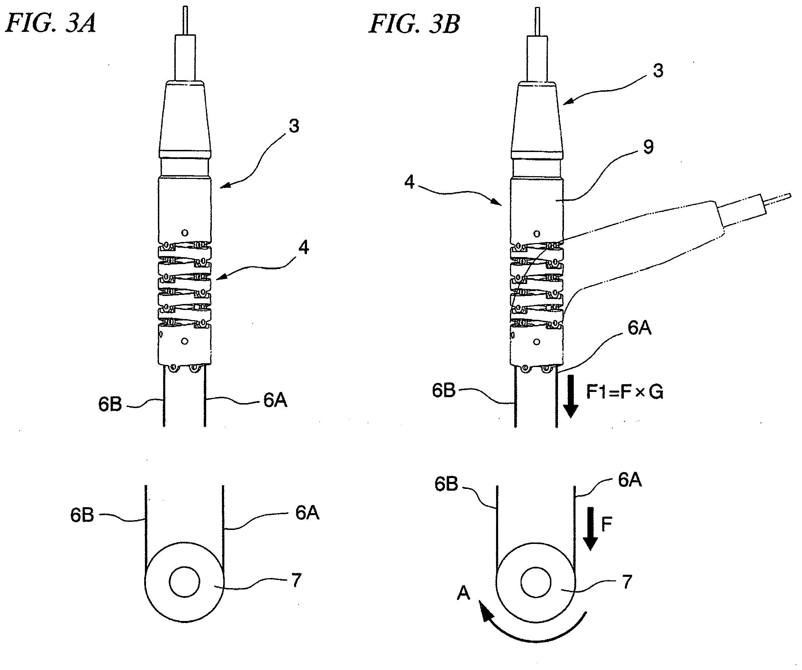

FIGS. 3A and 3B show an example of conventional art provided at the arm section 3 having a common bending mechanism. The proximal ends of the pair of wires 6A and 6B extending from the bending section 4 as shown in FIG. 3A are fixed to the outer periphery of the pulley 7 shown as an example of an operation section. Therefore, for example, when a user rotates the pulley 7 in a direction indicated by an arrow A shown in FIG. 3B via an angle knob attached to the pulley 7 coaxially, the wire 6A is retracted proximally, and the main unit 9 having the distal end of the wire 6A fixed thereto is drawn. This results in causing the arm section 3 to bend as indicated by a phantom line shown in FIG. 3B.

FG, that is obtained based on F×G, indicates a force F1 input into the distal end of this state of wire 6A wherein F indicates a force applied to the wire 6A by rotating the pulley 7 and G indicates the transfer coefficient of the wire 6A. It should be noted that the force is not transferred to the distal end of the wire 6B since the rotation of the pulley 7 provides slack to the wire 6B.

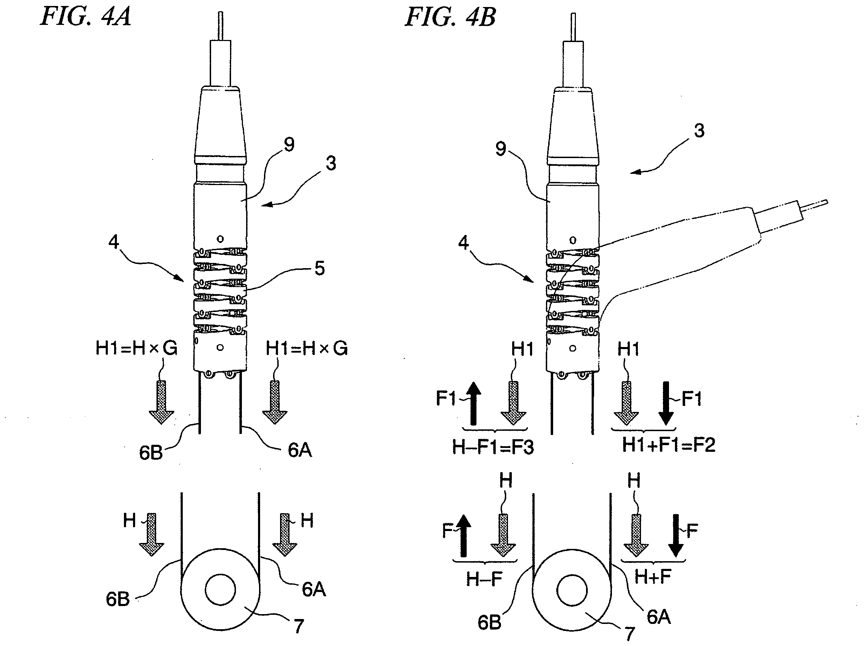

FIGS. 4A and 4B show the bending mechanism of the arm section 3 according to the present invention. As shown in FIG. 4A, the wires 6A and 6B in a previously-tension-applied state (the tension is hereinafter called a pre-tension) is connected to the main unit 9 and the pulley 7, and fixed thereto. Substantially the same magnitude of pre-tension is applied to the wires 6A and 6B respectively. HG, that is, obtained based on HxG indicates magnitude H1 of the pre-tension applied to the distal end of each wire wherein H indicates the magnitude of pre-tension in the vicinity of the pulley 7 and G indicates the transfer coefficient of the wires 6A and 6B similar to the aforementioned transfer coefficient. Therefore, equalized pre-tension in a neutral state, in which the pulley 7 is not operated, maintains the arm section 3 in a linear state since the pre-tension having the same magnitude of H1 is applied to the bending section 4 so that the pre-tensions face each other to place the axial line of the joint ring 5 therebetween.

Rotating this state of pulley 7 to apply a force having magnitude F to the wire 6A similar to the case of FIG. 3B provides additional pre-tension H, thereby providing a force H+F that acts on to the wire 6A in the vicinity of the pulley 7 as shown in FIG. 4B. H1+F1 obtained based on HG+FG indicates magnitude F2 of the force transferred to the distal end of the wire 6A.

This state of the wire 6B having pre-tension applied thereto and linking with the rotation of the pulley 7 is pushed out distally opposite to the wire 6A while releasing the pre-tension. Therefore, H-F indicates a force acting on the wire 6B in the vicinity of the pulley 7, and H1-F1 obtained based on HG-FG, indicates a force F3 transferred to the distal end of the wire 6B.

The following is a calculation for obtaining the magnitude of torque produced by the aforementioned force and acting to bend the bending section 4 of the arm section 3.

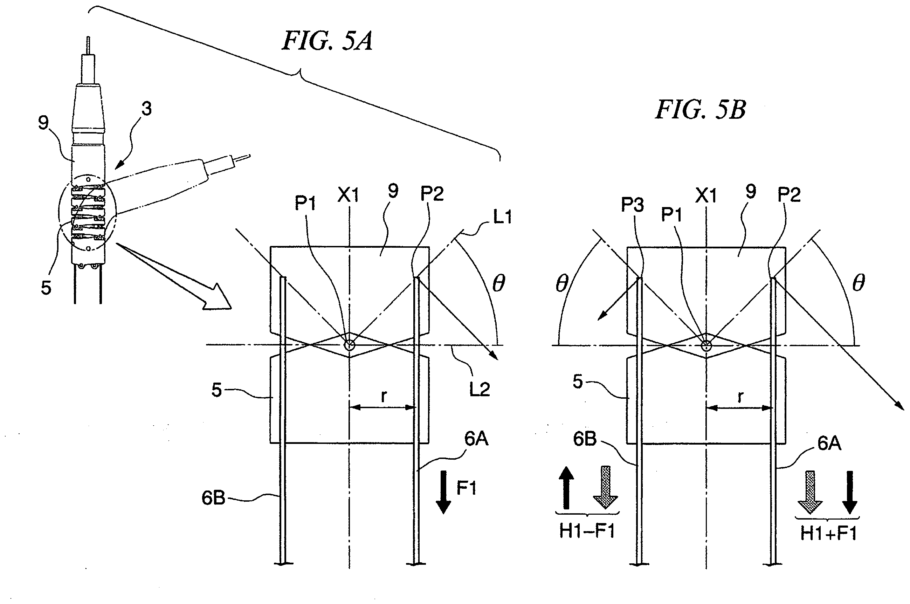

Torque T1 produced in the conventional example shown in FIG. 3B is F1cosθ×(r/cosθ)=F1r, wherein P1 indicates the position (that is, the rotational axis of the main unit 9) where the main unit 9 having the wires 6A and 6B of the arm section 3 connected thereto is connected to the adjacent joint ring 5; P2 indicates the position where the distal end of the wire 6A subject to retraction is fixed to the main unit 9; θ indicates the angle defined by a line segment L1 connecting the connection position P1 and the fixture position P2 and a line L2 passing through the connection position P1 and orthogonal to an axial line X1 so that the connection position P1 is positioned on the axial line X1 of the joint ring 5 in plan view; and r indicates the distance between the connection position P1 and the wire 6A as shown in FIG. 5A.

Torque T2 occurred by operating the arm section 3, as shown in FIG. 4B, having a bending tube structure as shown in FIG. 5B according to the present invention is indicated by 2×F1r=[H1 + F1]cosθ×(r/cosθ) - [H1 - F1]cosθ×(r/cosθ) wherein a connection position P3, indicating the point where the wire 6B is connected with the main unit 9, is symmetric relative to the connection position P1 with respect to the axial line X1.

That is, the bending tube according to the present invention produces bending torque substantially twice as significant as that of the conventional bending tube when a bending operation inputs the same magnitude of force into the pulley 7. Therefore, the user can conduct the bending operation with a force having substantially half the conventionally necessary magnitude.

In addition, fewer limitations associated with the configuration (e.g. the size of the operation section) can increase the design freedom for medical apparatuses adapting the bending tube since half the conventional operation degree can achieve substantially the same degree of stroke (bending range) in the arm section.

It should be noted that the magnitude H of the applied pre-tension can be set appropriately as long as the transfer member such as the wires 6A and 6B, for example, can endure the pre-tension. However, the force F1 applied to the distal end of the wire (i.e., the wire 6B in the example shown in FIG. 4B) opposite the wire retracted by operating the operation section and exceeding the pre-tension H1 applied to the distal end provides slack to the wire, thereby preventing the force from being transferred to the distal end of the wire; therefore, it is difficult to obtain the aforementioned effect. Therefore, it is preferable to set the maximum value of force F applied by a bending operation, and then set the magnitude of the pre-tension H that is more significant than the maximum value of the force F.

### [Second Embodiment]

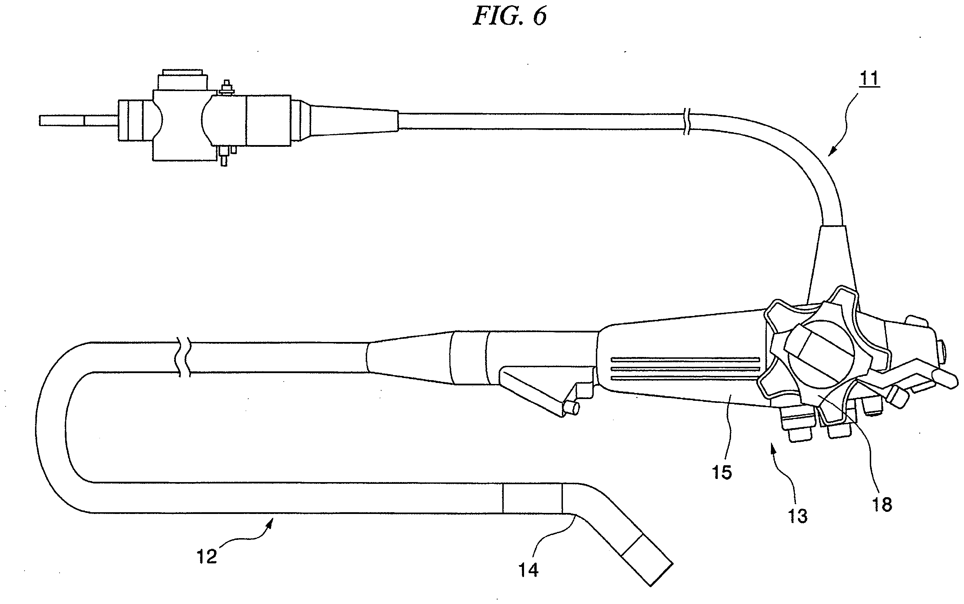

A second embodiment of the present invention will be explained next with reference to FIGS. 6 to 8. A bending tube of the present embodiment is different from the bending tube of the aforementioned first embodiment because the bending tube of the present embodiment is provided with a switching mechanism for switching the pre-tension, i.e. applying or releasing the pre-tension.

A medical apparatus shown in FIG. 6 is an endoscope 11 provided with the bending tube of the present embodiment. The endoscope 11 has an elongated flexible insertion section 12 and an operation section 13 attached to the proximal end of the insertion section 12.

The bending tube of the present embodiment is a insertion section 12 having a bending section 14 attached to the distal end thereof so that the bending tube can follow a serpentine path such as digestive tract, for example, when inserted into a body cavity. The structure of the bending section 14 is basically the same as that of the bending section 4 of the first embodiment. That is, a plurality of joint rings, not shown in the drawing, are connected in the axial line direction, and a pair of wires serving as the transfer member and having an end section fixed to the main unit having the most distal joint ring connected thereto are inserted into each joint ring and extend to the operation section 13.

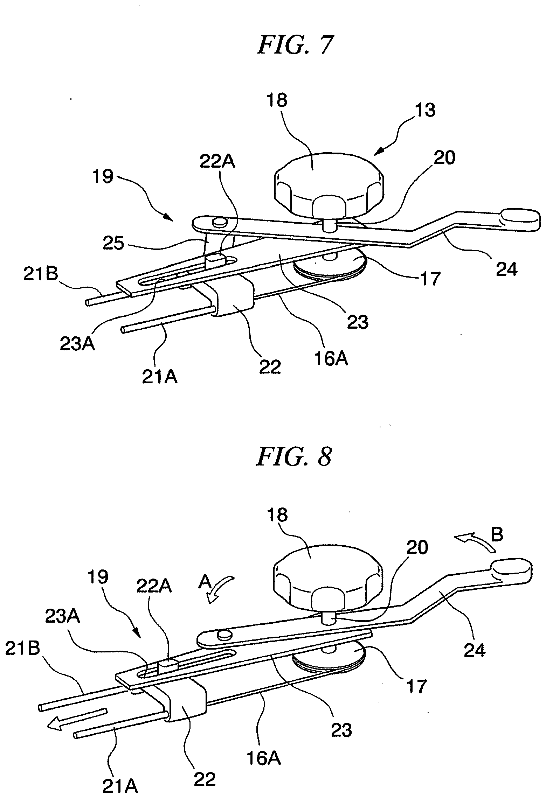

FIG. 7 shows the mechanism of the operation section 13 in isometric view. In order to show the interior of the operation section 13, a cover 15, for example, shown in FIG. 6 is omitted. The operation section 13 is provided with: a pulley 17 having a pair of wires 16A and 16B, not shown in the drawings, connected thereto, and extending from the bending section 14; an angle knob 18 for conducting a rotational operation of the pulley 17; and a switching mechanism 19 for switching on or off the pre-tension applied to the wires 16A and 16B.

The proximal ends of the wires 16A and 16B are connected and fixed to the outer periphery of the pulley 17 in substantially the same form of the aforementioned arm section 3, and rotational operation to the pulley 17 can retract one of the wires 16A and 16B proximally and bend the bending section 14.

Rotating the angle knob 18 attached to the pulley 17 coaxially via the shaft 20 allows the pulley 17 to rotate.

The switching mechanism 19 is provided with: coils 21A and 21B having the wires 16A and 16B inserted therethrough respectively; a coil base 22 having end sections of the coils 21A and 21B fixed thereto; a guide 23 for guiding the movement of the coil base 22; a lever 24 subject to a user's operation; and a link 25 connecting the lever 24 to the coil base 22.

A predetermined clearance reserved between the coils 21A and 21B and the wires 16A and 16B subject to insertion respectively allows the wires 16A and 16B to freely slide in the coils 21A and 21B respectively. Each distal end of the coils 21A and 21B is fixed to the joint ring of the bending section 14, and each proximal end thereof is connected and fixed to the coil base 22.

The coil base 22 has a projection 22A. The projection 22A is inserted through an elongated hole 23A of the guide 23 attached to the shaft 20. Sliding the projection 22A along the elongated hole 23A causes the guide 23 to guide the movement of the coil base 22.

The lever 24 attached to the shaft 20 is freely rotatable, and a first end section of the link 25 attached to one of the end sections of the lever 24 is freely rotatable. A second end section of the link 25 attached to the coil base 22 is freely rotatable.

Operations in using the endoscope 11 having the aforementioned configuration will be explained.

As shown in FIG. 7, the state of the projection 22A of the coil base 22 positioned proximally in the elongated hole 23A of the guide 23 indicates an "off" state in which pre-tension is not applied to the wires 16A and 16B, and the wires 16A and 16B have slack. Therefore, the bending section 14 can bend flexibly by applying an external force thereto. It should be noted that a user can bend the bending section 14 by conducting rotational operation to the angle knob 18 in the "off" state.

The user in an attempt to obtain an "on" state of the wires 16A and 16B having pre-tension applied thereto conducts rotational operation to the lever 24 in the direction indicated by an arrow B as shown in FIG. 8. Accordingly, as shown in FIG. 8, the coil base 22 moves along the elongated hole 23A distally relative to the wires 16A and 16B. The movement of the coil base 22 causes the distance between the coil base 22 and the pulley 17 to increase, thereby causing the distance between the wires 16A and 16B between the proximal end of the coil base 22 and the pulley 17 to increase by the same length. This results in applying pre-tension to the wires 16A and 16B corresponding to the movement length of the coil base 22.

The user, upon rendering the wires 16A and 16B to be in the "on" state, can conduct operation to the bending section 14 preferably with fewer degrees of operation to the angle knob 18, for example, similarly to the arm section 3 of the first embodiment.

The endoscope 11 according to the present embodiment having the switching mechanism 19 provided to the operation section 13 can provide preferable operation by switching pre-tension while insertion to the body cavity is subject to the "off" state and treatment is subject to the "on" state.

In addition, the shape of the bending section 14 can be fixed by applying pre-tension to the wires 16A and 16B. Accordingly, the distal end section of the insertion section 12 having the bending section 14 provided thereto, which if applied an external force, can be maintained, and the visual field of the endoscope 11 can be maintained desirably.

### [Third Embodiment]

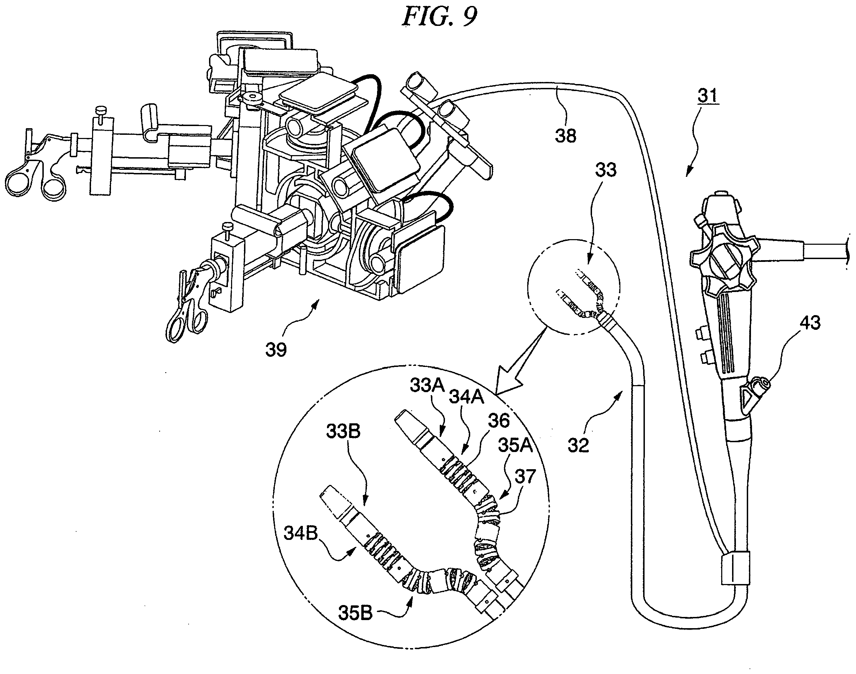

A third embodiment of the present invention will be explained next with reference to FIGS. 9 to 14. A bending tube of the present embodiment is different from the bending tube of each aforementioned embodiment because switching on the pre-tension and off can be conducted simultaneously with other bending operations.

A medical apparatus shown in FIG. 9 is an endoscope 31 provided with the bending tube of the present embodiment. An insertion section 32 and two arms 33A and 33B of an arm section 33 in the treatment endoscope 31 have structures substantially the same as the insertion section 2 and the arm section 3 of the first embodiment respectively.

The bending tube of the present embodiment, (i.e., the arms 33A and 33B) have first bending sections 34A and 34B having the basic structure substantially the same as that of the bending section 4 of the arm section 3 and second bending sections (fixed bending sections) 35A and 35B provided proximally relative to the first bending sections 34A and 34B respectively.

Two pairs of two wires, not shown in the drawing, are attached to the substantial cylindrical joint rings 36A of the first bending sections 34A and 34B respectively so that each pair of wires face each other to place the axial line of the joint rings 36 therebetween, and the wires are shifted by a phase of 90 degrees. Accordingly, conducting appropriate retracting operations to four wires allows the arms 33A and 33B to bend in four directions.

The second bending sections 35A and 35B are mechanisms capable of bending and fixing thereof to facilitate operation for the treatment instruments inserted through the arms 33A and 33B in a treatment which will be explained later so that the distance of the axial lines of the arms 33A and 33B increases more significantly as shown in FIG. 9. Wires are fixed and inserted in joint rings 37 of the second bending sections 35A and 35B respectively by the same method as that for the first bending sections 34A and 34B. The wires extending from the first bending sections 34A and 34B and the second bending sections 35A and 35B further extend to a first operation section 39, which will be explained later, through a link sheath 38.

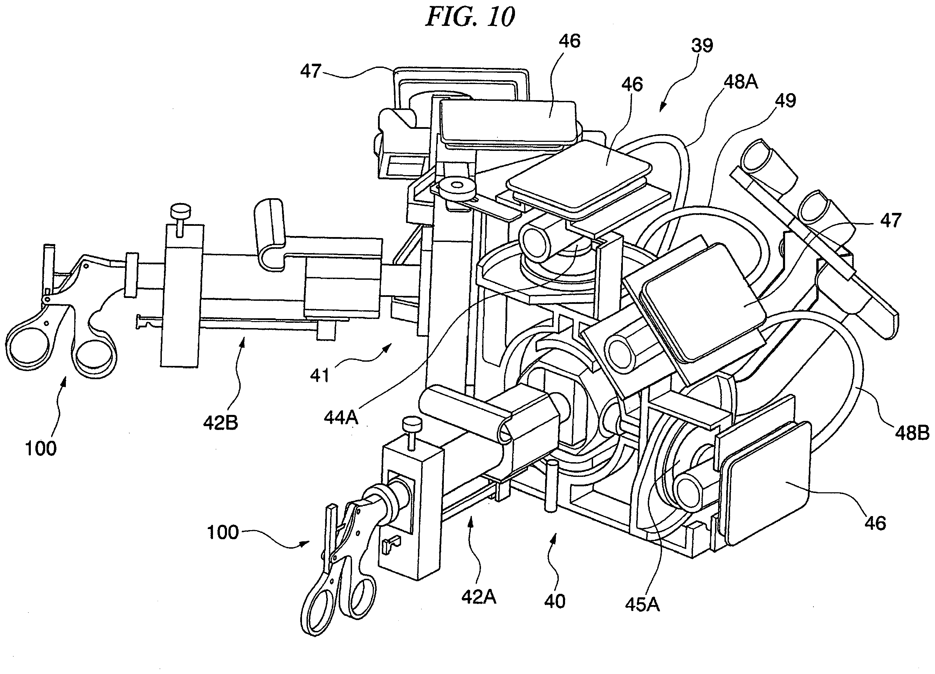

FIG. 10 is an enlarged view of the operation section 39. The operation section 39 for operating the arm section 33 is provided with a first operation unit 40 for operating the arm 33A and a second operation unit 41 for operating an arm 33B.

Operation sticks 42A and 42B subject to the user's operation are attached to the operation units 40 and 41 respectively. Each of the operation sticks 42A and 42B having the same structure has a channel for inserting the treatment instrument 100 therethrough. Each channel is connected to a forceps port 43 (see FIG. 9) via a tube etc. not shown in the drawings and communicates with the operation channel, not shown in the drawings, in the insertion section 32 respectively.

First operation shafts 44A and 44B and second operation shafts 45A and 45B, not shown in the drawing, are, respectively attached to the operation sticks 42A and 42B, not shown in the drawing. The first operation shafts 44A and 44B make rotation linked with vertical operation by the operation sticks 42A and 42B. The second operation shafts 45A and 45B make rotation linked with horizontal operation by the operation sticks 42A and 42B. The wires extending through the link sheath 38 for operating the first bending sections 34A and 34B are attached to the first operation shafts, and to the second operation shafts of the operation units 40 and 41 respectively and detachably. A pair of the wires are connected to each first wire unit 46.

This allows the treatment instrument 100 to be inserted into the operation sticks 42A and 42B and protrude the treatment instrument 100 from the distal end of the arm section 33, thereby, moving a treatment mechanism provided to the distal end of the treatment instrument 100 vertically and horizontally by operating the operation sticks 42A and 42B vertically and horizontally for operating the arm section 33.

In addition, wires for operating the second bending sections 35A and 35B are connected to a second wire unit 47 having a structure substantially the same as that of the first wire unit 46 and attached to the operation units 40 and 41 respectively.

First retraction wires 48A and 48B for applying pre-tension to the wires connected to the first wire unit 46 extend from the first operation shaft 44A and the second operation shaft
45A respectively. A second retraction wire 49 for retracting the second wire unit 47 proximally extends from the second wire unit 47. It should be noted that the second operation unit 41, not shown in the drawings, has a similar configuration.

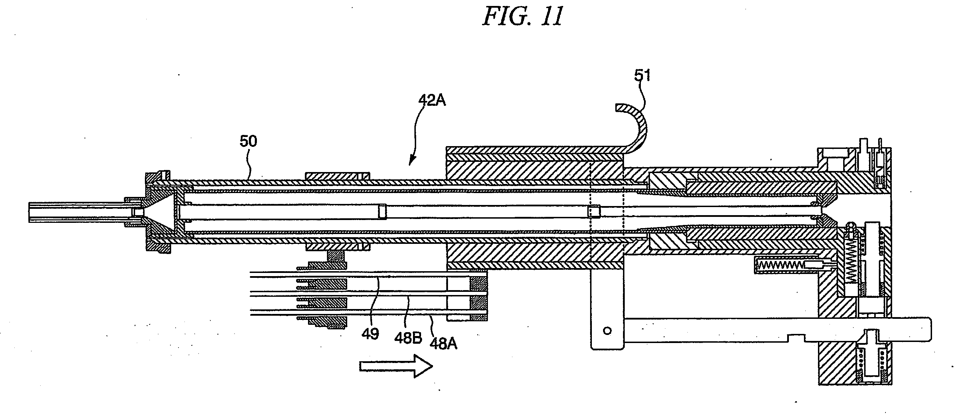

FIG. 11 shows the operation stick 42A in cross sectional view. As shown in FIG. 11, the first retraction wires 48A and 48B and the second retraction wire 49 are connected to a slide lever 51 attached to a main unit 50 of the operation stick 42A and fixed there. The slide lever 51 is capable of freely sliding on the main unit 50. Therefore, these three wires can be retracted by sliding the slide lever 51 to be attracted proximally relative to the main unit 50.

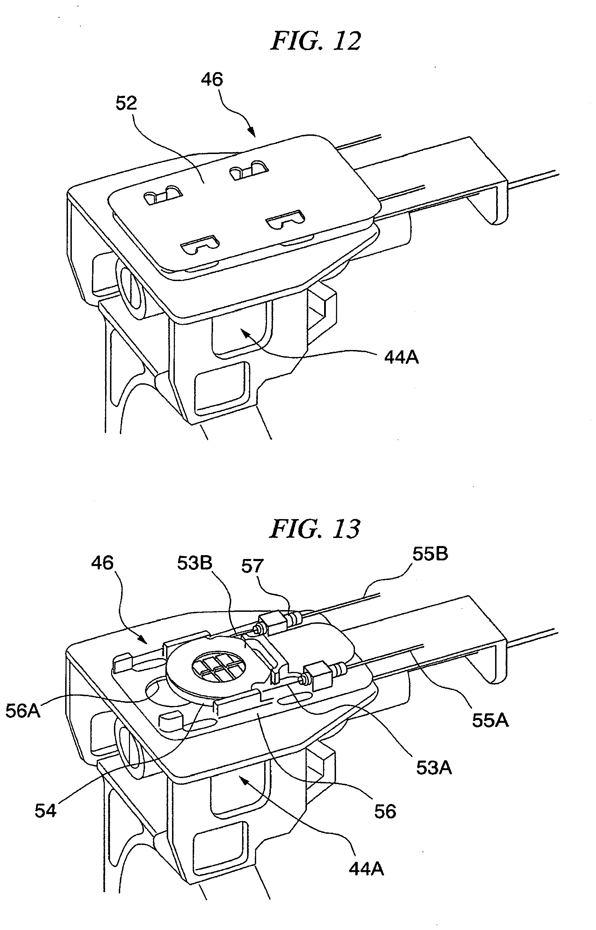

FIG. 12 shows the first wire unit 46 attached to the first operation shaft 44A in an enlarged view. A cover 52 covers the upper surface of the first operation shaft 44A.

FIG 13 shows the cover 52 in a removed state. The proximal ends of wires 53A and 53B extending from the first bending section 34A are connected to the pulley 54 and fixed thereto. The pulley 54 has a rotation shaft, not shown in the drawings, which extends downward. The rotation shaft is inserted into the first operation shaft 44A coaxially.

The wires 53A and 53B are inserted into coils 55A and 55B respectively. The distal ends of the coils 55A and 55B are fixed to the joint ring 36 of the first bending section 34A, and the proximal ends thereof are fixed to a coil base 57 attached to a base 56 of the first wire unit 46. The rotation shaft of the pulley 54 is inserted into an elongated hole 56A provided on the base 56. It should be noted that the second operation shaft [[44]]45A has a similar condition.

Operations in using the treatment endoscope 31 having the aforementioned configuration will be explained.

The user firstly inserts the insertion section 32 into a body cavity of a patient etc. The second bending section in this state of the arm section 33 is not bent and two arms 33A and 33B are set in a linear state for facilitating insertion. Also, the wires of the first bending sections 34A and 34B are in the off-state in which pre-tension is not applied thereto so that the arms 33A and 33B can bend flexibly in accordance with the shape of an organ, for example, winding in the body cavity. The distal end of the treatment endoscope 31 is moved in this way to a treatment object site.

Subsequently, the user inserts appropriately-selected treatment instruments into the operation sticks 42A and 42B and projects the distal ends thereof from the arm section 33. Subsequently, the slide lever 51 of each operation stick is operated to be attracted proximally.

This operation causes the second retraction wire 49 to be retracted proximally and causes the second wire unit 47, which is not attached to the operation shaft, to move proximally. Accordingly, the wires connected to the second wire unit 47 are retracted, and as shown in FIG. 9, the second bending sections 35A and 35B of the arm section 33 are fixed in a bent state; thus, a condition facilitating a treatment using a treatment instrument is provided.

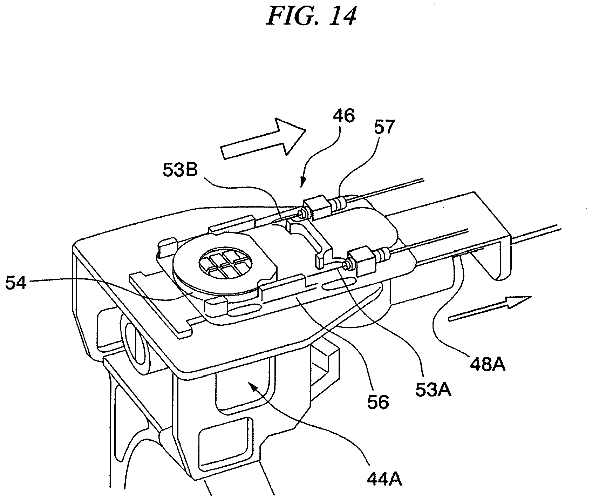

First retraction wires 48A and 48B in this state are retracted simultaneously. Accordingly, the base 56 of the first wire unit 46 moves distally in the first operation shaft 44A and the second operation shaft 45A as indicated by an arrow shown in FIG. 14. However, the pulley 54 does not move since the rotation shaft of the pulley 54 is inserted into the first operation shaft 44A and the second operation shaft 45A through the elongated hole 56A. Therefore, the base 56 and the coil base 57 move in the distal end direction of the wires 53A and 53B relative to the pulley 54. Accordingly, pre-tension is applied to the wires 53A and 53B based on a principle equivalent to that of the second embodiment.

The treatment endoscope 31 according to the present embodiment can achieve similar effects of the medical apparatus provided with the bending tube according to each aforementioned embodiment.

Also, since a first retraction wire for applying pre-tension to wires connected to the first bending sections 34A and 34B and a second retraction wire for bending and fixing the second bending sections 35A and 35B in a state for facilitating a treatment are connected together to the slide lever 51 provided to the operation stick, a mere retracting operation of the slide lever 51 can conduct the two operations simultaneously.

Therefore, since the second bending sections 35A and 35B which are about to be inserted into a body cavity are in a non-fixed state in the treatment endoscope 31, and since pre-tension is not applied to the first bending sections 34A and 34B, the two arms, i.e., the two bending tubes of the arm section 33 are in a freely and flexibly deformable state; therefore, insertability can be enhanced. On the other hand, the second bending sections 35A and 35B are fixed in a state facilitating a treatment when the treatment is conducted, and simultaneously, pre-tension is applied to the first bending sections 34A and 34B; therefore, an easily operable state is achieved. Since the user can switch these two states easily by operating the slide lever 51, an easily operable treatment endoscope can be configured.

It should be noted that the present embodiment explained with reference to the example which adopts the bending tube of the present invention at only the arm section 33 may be replaced by a configuration in which the bending tube of the present invention is adopted in the insertion section 32 similarly to the insertion section 12 of the endoscope of the second embodiment. In this case, when a tissue retraction etc. is conducted by using a treatment instrument, for example, inserted into the arm section 33, a more desirable retraction, for example, can be conducted by applying pre-tension to the insertion section 32 and preventing deformation thereof.

Although the present invention has been described with respect to its preferred embodiments, the present invention is not limited to the embodiments described above. The configuration of the present invention allows for addition, omission, substitution and further modification.

For example, the aforementioned each embodiment explained with reference to the example in which two proximal ends of two wires are fixed to a pulley may be replaced by a configuration in which a wire is wound around a pulley and two ends thereof are fixed to a bending section respectively as long as the wire fixed to the pulley is rotatable unitarily with the pulley.

Also, another mechanism may be used in place of wires and a pulley since the effect of the present invention can be obtained if one of the wires is retracted and the other wire makes linked movement in the reverse direction. For example, the linked movement may be achieved by using a chain and a sprocket, and alternatively, a rack-and-pinion mechanism may make a linked movement of a pair of wires wherein the proximal ends of the wires are fixed to the rack.

Furthermore, the aforementioned each embodiment explained with reference to the example in which pre-tension is applied to a transfer member, e.g. a wire by moving a coil having the wire inserted therethrough in the axial line direction may be replaced by a configuration in which pre-tension is applied to a transfer member by moving an operation section (e.g. a pulley) proximally having the proximal end of a transfer member fixed thereto.

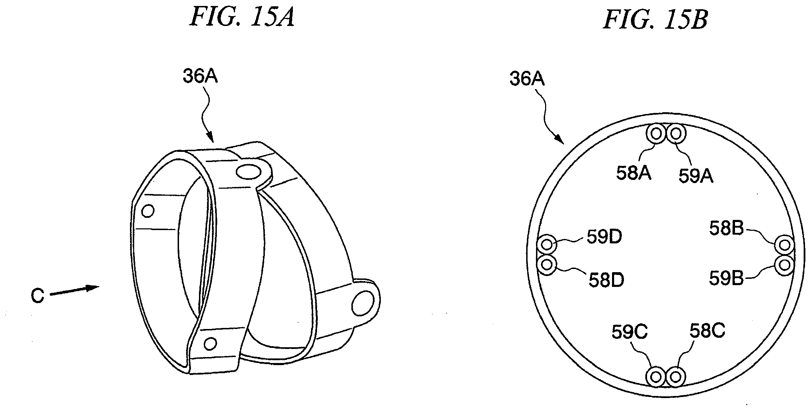

In addition, like a modified examples shown in FIG. 15A and 15B, insertion tubes 59A to 59D having wires for applying pre-tension inserted therethrough may be attached to a joint ring 36A of a bending section separately from insertion tubes 58A to 58D having wires used for operation inserted therethrough, and the wires for operating the bending section may be disposed adjacent to the wires for applying the pre-tension. Accordingly, although it is not possible to decrease bending operation amount or decrease a force required for the bending operation similarly to the bending tube of the aforementioned each embodiment, a desirable bending state of a bending section obtained by operating wires used for operation can be maintained by retracting the wires in order to apply the pre-tension.

Therefore, the bending tube can be fixed in an arbitrary bending state, and operation can be conducted more easily. Furthermore, in a case of retraction, for example, of a tissue by using a treatment instrument inserted into the bending tube, more reliable retraction can be conducted since the deformation of the bending tube is prevented.

Also, the aforementioned each embodiment is not limited to the medical apparatus explained with reference to the example in which the medical apparatuses provided with the bending tube of the present invention are a treatment endoscope and an endoscope. Therefore, anything that conducts bending operation can be adopted to every medical apparatus including various common treatment instrument.

The present invention is not limited to the above descriptions but is limited only by the appended claims.

## Claims

1. A bending tube, provided with a bending section (4) capable of bending, the bending tube comprising:
a main unit (9) having a lumen;
a pair of transfer members (6A, 6B) each inserted into the bending section (4), distal ends of the transfer members (6A, 6B) being connected to a distal end of the bending section (4) or the main unit (9) disposed distally relative to the bending section;
an operation section having proximal ends of the pair of transfer members (6A, 6B), the operation section being configured so that a retracting operation for retracting one of the pair of transfer members (6A, 6B) proximally causes the other one of the pair of transfer members (6A, 6B) to be linked and pushed out distally;
a switching mechanism (19) fixed on the transfer members disposed between the operation section and a distal end of the transfer members and configured to be capable of applying and releasing the pre-tension relative to the pair of transfer members; **characterized in that**
a predetermined magnitude of pre-tension is applicable to the pair of transfer members (6A, 6B) by enlarging a distance between the operation section and the switching mechanism (19) when the transfer members (6A, 6B) are in a neutral state, and
the predetermined magnitude of the pre-tension is set so as to apply tension to the transfer members (6A, 6B) constantly without recourse to a deformation of the bending section (4) due to the bending action thereof.

2. The bending tube according to Claim 1, wherein the switching mechanism releases the pre-tension by moving the operation section in a direction toward the distal ends of the transfer members.

3. The bending tube according to Claim 1, wherein the operation section has a pulley (7), and the proximal ends of the pair of transfer members (6A, 6B) are connected to an outer periphery of the pulley (7).

4. A medical apparatus (11) comprising the bending tube according to Claim 1.

5. A medical apparatus comprising:
an elongated flexible insertion section (32); and
the bending tube (33A, 33B) according to Claim 1 attached to a distal end of the insertion section (32), wherein
the bending tube (33A, 33B) has a second transfer member inserted therethrough and is provided at a position different from the bending section (34A, 34B, 35A, 35B), the bending tube (33A, 33B) has a fixed bending section which is bent and fixed to a predetermined shape by operating the second transfer member, and
the proximal end of the second transfer member are connected to the switching mechanism (39), and when the pre-tension is applied to the second transfer member by the switching mechanism (39), the fixed bending section is linked to be bent and fixed to a predetermined shape.

## Patentansprüche

1. Biegerohr, das mit einem Biegeabschnitt (4) versehen ist, der gebogen werden kann, wobei das Biegerohr umfasst:
eine Haupteinheit (9) mit einem Lumen;
ein Paar Transferelemente (6A, 6B), die jeweils in den Biegeabschnitt (4) eingesetzt sind, wobei die distalen Enden der Transferelemente (6A, 6B) mit einem distalen Ende des Biegeabschnitts (4) oder der Haupteinheit (9), die bezogen auf den Biegeabschnitt distal angeordnet ist, verbunden sind;
einen Bedienungsabschnitt, der die proximalen Enden des Paares Transferelemente (6A, 6B) aufweist, wobei der Bedienungsabschnitt derart ausgelegt ist, dass eine Zurückziehbedienung zum proximalen Zurückziehen von einem des Paares Transferelemente (6A, 6B) bewirkt, dass das andere des Paares Transferelemente (6A, 6B) angekuppelt und distal herausgeschoben wird;
einen Schaltmechanismus (19), der an den Transferelementen befestigt ist und zwischen dem Bedienungsabschnitt und einem distalen Ende des Transferelements angeordnet und dazu ausgelegt ist, die Vorspannung bezogen auf das Paar Transferelemente anzulegen und zu lösen; **dadurch gekennzeichnet, dass**
ein vorbestimmter Vorspannungsbetrag durch Vergrößern eines Abstands zwischen dem Bedienungsabschnitt und dem Schaltmechanismus (19) auf das Paar Transferelemente (6A, 6B) aufgebracht werden kann, wenn die Transferelemente (6A, 6B) in einem neutralen Zustand sind, und
der vorbestimmte Vorspannungsbetrag derart festgelegt ist, dass ohne Verzicht auf eine Verformung des Biegeabschnitts (4) aufgrund seines Biegevorgangs konstant eine Spannung auf die Transferelemente (6A, 6B) aufgebracht wird.

2. Biegerohr nach Anspruch 1, wobei der Schaltmechanismus die Vorspannung durch Bewegen des Bedienungsabschnitts in Richtung der distalen Enden der Transferelemente löst.

3. Biegerohr nach Anspruch 1, wobei der Bedienungsabschnitt eine Riemenscheibe (7) aufweist, und die proximalen Enden des Paares Transferelemente (6A, 6B) mit einem Außenumfang der Riemenscheibe (7) verbunden sind.

4. Medizinische Vorrichtung (11), umfassend das Biegerohr nach Anspruch 1.

5. Medizinische Vorrichtung, umfassend:
einen länglichen flexiblen Einsetzabschnitt (32); und
das Biegerohr (33A, 33B) nach Anspruch 1, das an einem distalen Ende des Einsetzabschnitts (32) angebracht ist, wobei
das Biegerohr (33A, 33B) ein zweites Transferelement aufweist, das darin eingesetzt ist, und in einer Position vorgesehen ist, die sich von dem Biegeabschnitt (34A, 34B, 35A, 35B) unterscheidet, wobei das Biegerohr (33A, 33B) einen festen Biegeabschnitt aufweist, der gebogen und in einer vorbestimmten Form durch Bedienen des zweiten Transferelements fixiert wird, und
das proximale Ende des zweiten Transferelements mit dem Schaltmechanismus (39) verbunden ist, und wenn die Vorspannung durch den Schaltmechanismus (39) an das zweite Transferelement angelegt wird, wird der feste Biegeabschnitt angekuppelt, um gebogen und in einer vorbestimmten Form fixiert zu werden.

## Revendications

1. Tube de flexion, comprenant une section de flexion (4) capable de fléchir, le tube de flexion comprenant :
une unité principale (9) ayant une lumière ;
une paire d'éléments de transfert (6A, 6B) insérés chacun dans la section de flexion (4), des extrémités distales des éléments de transfert (6A, 6B) étant reliées à une extrémité distale de la section de flexion (4) ou à l'unité principale (9) disposée de manière distale par rapport à la section de flexion ;
une section d'opération ayant des extrémités proximales de la paire d'éléments de transfert (6A, 6B), la section d'opération étant configurée de telle sorte qu'une opération de rétractation pour rétracter l'un de la paire d'éléments de transfert (6A, 6B) amène de manière proximale l'autre de la paire d'éléments de transfert (6A, 6B) à être relié et poussé vers l'extérieur de manière distale ;
un mécanisme de commutation (19) fixé sur les éléments de transfert, disposé entre la section d'opération et une extrémité distale des éléments de transfert et configuré pour être capable d'appliquer et de libérer la pré-tension par rapport à la paire d'éléments de transfert ; **caractérisé par le fait que**
une grandeur prédéterminée de pré-tension est applicable à la paire d'éléments de transfert (6A, 6B) par agrandissement d'une distance entre la section d'opération et le mécanisme de commutation (19) lorsque les éléments de transfert (6A, 6B) sont dans un état neutre, et
la grandeur prédéterminée de la pré-tension est définie de façon à appliquer une tension aux éléments de transfert (6A, 6B) de manière constante sans avoir recours à une déformation de la section de flexion (4) due à l'action de flexion de celle-ci.

2. Tube de flexion selon la revendication 1, dans lequel le mécanisme de commutation libère la pré-tension par déplacement de la section d'opération dans une direction vers les extrémités distales des éléments de transfert.

3. Tube de flexion selon la revendication 1, dans lequel la section d'opération a une poulie (7), et les extrémités proximales de la paire d'éléments de transfert (6A, 6B) sont reliées à une périphérie externe de la poulie (7).

4. Appareil médical (11) comprenant le tube de flexion selon la revendication 1.

5. Appareil médical comprenant :
une section d'insertion souple allongée (32) ; et
le tube de flexion (33A, 33B) selon la revendication 1, attaché à une extrémité distale de la section d'insertion (32),
le tube de flexion (33A, 33B) ayant un second élément de transfert inséré à travers celui-ci et étant disposé à une position différente de la section de flexion (34A, 34B, 35A, 35B), le tube de flexion (33A, 33B) ayant une section de flexion fixe qui est fléchie et fixée dans une forme prédéterminée par actionnement du second élément de transfert, et
l'extrémité proximale du second élément de transfert étant reliée au mécanisme de commutation (39) et, lorsque la pré-tension est appliquée au second élément de transfert par le mécanisme de commutation (39), la section de flexion fixe étant reliée pour être fléchie et fixée dans une forme prédéterminée.
